# EUROPEAN PATENT APPLICATION

(11) **EP 3 031 809 A1**
(43) Date of publication of application: **15.06.2016**
(21) Application number: 14382501.6
(22) Date of filing: 09.12.2014
(51) Int. Cl.: C07D 498/04, A61K 31/424, A61P 35/00

(54) **2-phenyl-7,7a-dihydro-3aH-pyran[3,4-d]oxazol-6(4H)-ones**

(71) Applicant: CONSEJO SUPERIOR DE INVESTIGACIONES CIENTIFICAS, 28006 Madrid (ES)
(72) Inventor: Sánchez Puelles, José María, 28040 Madrid (ES); Sánchez Sancho, Francisco, 28006 Madrid (ES)
(74) Representative: Pons Ariño, Angel

(57) **Abstract**

Derivatives of 2-phenyl-7,7a-dihydro-3a*H*-pyrano[3,4-d]oxazole-6(4*H*)-one of formula I where the meanings for the substituents are those listed in the description. These compounds are useful as inhibitors of HIF-2α and SOX-2.

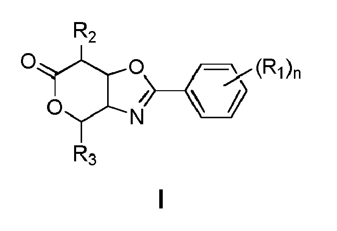

## Description

This invention refers to a new series of compounds derived from 2-phenyl-7,7a-dihydro-3a*H*-pyrano[3,4-d]oxazole-6(4*H*)-one, as well as the process for preparing them, to the pharmaceutical composition that includes them and to their use in therapy, particularly in cancer.

### STATE OF THE ART

Currently cancer is the main cause of death in many developed countries and will be a serious cause of morbidity and mortality in all regions of the world in the next decades. According to United Nations forecasts, cancer incidence will increase by 75% by 2030, mainly due to changes in the lifestyle of the population of less developed countries.

The presence of cancer stem cells (CSCs) is one of the main causes for the failure of current oncologic therapies, both at the level of prevention of tumour progression and in the appearance of recurrences (Cirenajwis H, et al., (2010) Anticancer Drugs 21(10):897-906). CSCs are recognised as a subtype of cancer cells that have phenotypic characteristics associated with embryonic stem cells or non-tumour progenitors, specifically with their ability to give rise to a heterogeneity of cell types that are found in a tissue such as a solid tumour. CSCs have diverse phenotypic characteristics but share the presence of markers associated with embryonic and progenitor cells. However, they also have other phenotypic characteristics that are not associated with stem cells and for this reason many authors believe that the more correct name for these cells should be tumour initiating cells (TICs) rather than CSCs. However, the scientific literature has already accepted the name of CSCs to designate the cellular subtype responsible for initiation and expansion of an oncological disease.

CSCs are, therefore, tumourigenic (tumour-forming), originators of tumours, compared to other tumour cells that do not have the potential to cause tumours but do increase the tumour burden by uncontrolled proliferation. CSCs are characterised by the processes of self-renewal, originating new CSCs, and of differentiation, giving rise to different types of tumour cells. CSCs subpopulation is more resistant to treatments with chemical or radiation therapies. The persistence of CSCs in tumours as an undifferentiated and resistant to treatment population is at the origin of recurrences and metastases. This identification as a resistant subtype and responsible for recurrences has motivated their importance as a therapeutic target in current cancer research.

The study of transcription factors induced by hypoxia (Hypoxia Inducible Factors, hereafter called HIFs) has progressed in the recent decades from their role in tumour angiogenesis to the aetiology of oncological disease, particularly in the case of the HIFα subunits. Many studies have demonstrated an essential role of HIFα in the maintenance of cellular dedifferentiation, proliferative ability and self-renewal, as well as in oncogenesis, treatment resistance, relapses and metastases (reviewed in Majmundar A., et al (2010) Mol Cell 40(2): 294-309). Recently, the existence of a clear relationship between different transcription factors that are activated in tissue hypoxia (HIFs) and the pathways controlling cell self-renewal such as SOX-2, OCT3/4, NANOG and miR312 was demonstrated, suggesting an essential role in oncogenesis through the generation or expansion of the CSCs (Mathieu J., et al. (2011) Cancer Res. 71(13):4640-52). In fact, one of the HIFα subunits is the regulator of two of these stem factors, SOX-2 and OCT4, which in turn interact directly or indirectly with the other factors mentioned to regulate cell differentiation. The interruption of the HIFs signalling pathway is therefore a pharmaceutical research field for developing drugs for cancer treatment and more recently by its importance in maintaining the CSCs. In this sense, several compounds are known that directly act on HIFs expression. However, these compounds do not act on already generated CSCs, so new compounds directly targeted against them are necessary. Thus, a family of compounds that modulate transcriptional activity of HIFα proteins, inhibiting expression of SOX-2, OCT4, NANOG and TGF-α has been published (Moreno-Manzano V, et al (2010) JBC 285 (2) 1333-1342; Rodríguez-Jiménez F.J. and Moreno-Manzano V. (2011) Cell Mol Life Sci 69(4):519-34), but these compounds were toxic.

Therefore it would be desirable to provide new compounds capable of inhibiting the expression of the HIF-2α subunit and/or the SOX-2 transcription factor, and particularly those that would be able to inhibit HIF-2α activity and be good drug candidates. Such compounds should have good activity in pharmacological tests, good absorption when administered, as well as being metabolically stable and having a favourable pharmacokinetic profile. In addition, the compounds must not be toxic and have tolerable and/or clinically manageable side effects at the highly effective dose.

### DESCRIPTION OF THE INVENTION

A first aspect of this invention refers to a compound with the formula I: 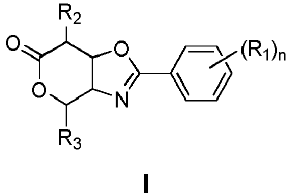 or a salt thereof where:
each R₁ independently represents alkyl C₁₋₁₂, alkenyl C₂₋₁₂, alkynyl C₂₋₁₂, alkyl Cy₁-C₁₋₄, alkenyl Cy₁-C₂₋₄, alkynyl Cy₁-C₂₋₄, -OR₄, -SR₄ or -NR₅R₅, where each alkyl C₁₋₁₂, alkenyl C₂₋₁₂, alkynyl C₂₋₁₂, alkyl Cy₁-C₁₋₄, alkenyl Cy₁-C₂₋₄ and alkynyl Cy₁-C₂₋₄ are independently and optionally replaced by one or more halogen, -O(alkyl C₁₋₄), -S(alkyl C₁₋₄), -NH(alkyl C₁₋₄) or -N(alkyl C₁₋₄)₂ groups;
R₂ represents hydrogen, alkyl C₁₋₄, -OR₄, -SR₄ or -NR₅R₅;
R₃ represents hydrogen or alkyl C₁₋₄;
n represents from 0 to 5;
each R₄ independently represents hydrogen, alkyl C₁₋₁₂, alkenyl C₂₋₁₂, alkynyl C₂₋₁₂, alkyl Cy₁-C₁₋₄, alkenyl Cy₁-C₂₋₄ or alkynyl Cy₁-C₂₋₄;
each R₅ independently represents hydrogen, alkyl C₁₋₄, -COR₆, -CONR₆R₆, -CO₂R₆ or -SO₂R₆;
or two R₅ groups may be bound on the same N atom forming a 5 or 6 member ring with the N atom, which may additionally contain one or two heteroatoms selected from N, S and O and are optionally substituted by one or more alkyl C₁₋₄ groups;
   each R₆ independently represents hydrogen, alkyl C₁₋₁₂, alkenyl C₂₋₁₂, alkynyl C₂₋₁₂, alkyl Cy₁-C₁₋₄, alkenyl Cy₁-C₂₋₄, alkynyl Cy₁-C₂₋₄;
Cy₁ represents phenyl or a saturated, partially unsaturated or aromatic 5 or 6 member heterocyclic ring linked to the rest of the molecule through any available C or N atom, each of which can be optionally fused to a saturated, partially unsaturated or aromatic 5 or 6 member heterocyclic ring, where Cy₁ may contain from 1 to 4 heteroatoms in total selected from N, O and S, where one or more of the available C or N atoms in Cy₁ are optionally oxidised forming CO, SO or SO₂ groups and where Cy₁ is optionally substituted by one or more R₇;
   R₇ represents alkyl C₁₋₄, halogen, -CN, -OR₈, -SR₈, -NR₈R₈; and
each R₈ independently represents hydrogen or alkyl C₁₋₄.

This invention also refers to the salts and solvates of the compounds of formula **I**. Therefore, any formula in this document is intended to represent hydrates, solvates and polymorphs of such compounds and mixtures of them.

Some of the compounds of formula **I** may have chiral centres, which may give rise to various stereoisomers. This invention refers to all the individual stereoisomers as well as to their mixtures.

The compounds of formula I are modulators of the hypoxia inducible transcription factor (HIF), particularly of HIF-2α and/or the transcription factor SOX-2 and can therefore be useful in the treatment of any disease mediated by this factor such as, but without being limited to, cancer.

Another aspect of this invention refers to a compound of formula **I** as previously described for its use in therapy.

Another aspect of this invention refers to a pharmaceutical composition that comprises a compound of formula **I** as described above and at least one pharmaceutically acceptable excipient.

Another aspect of this invention refers to a compound of formula **I** as previously defined or a pharmaceutically acceptable salt thereof for its use in the treatment of a disease involving modulation of the Hypoxia Inducible transcription Factor (HIF), preferably for the treatment of a disease that involves cells expressing one of the following markers: HIF-1α, HIF-2α, SOX-2, NR2F2, JAG1, PTEN, OCT4, NANOG, Klf-LF4, Notch, cMyc, miR21, miR205 or miR302; preferably for the treatment of a disease that involves modulation and/or inhibition of HIF-2α and/or SOX-2 such as, for example and without being limited to, cancer.

In another embodiment, the invention refers to a compound of formula **I** as previously defined or a pharmaceutically acceptable salt thereof for its use in the treatment of cancer, preferably for the treatment of breast cancer, brain cancer, head cancer, neck cancer, liver cancer, cervical cancer, colorectal cancer, endometrial cancer, gastric cancer, lung cancer, pancreatic cancer, ovarian cancer, prostate cancer and renal cancer, more preferably for the treatment of breast and brain cancer and still more preferably for the treatment of breast cancer.

Another aspect of this invention refers to a method for treating a disease involving modulation and/or inhibition of hypoxia inducible transcription factor (HIF) through the application of a compound of formula **I** as previously defined or a pharmaceutically acceptable composition as previously described. Preferably, the method for treating a disease involving modulation and/or inhibition of HIF-2α and/or SOX-2. More preferably, a method for the treatment of cancer and preferably cancers expressing biomarkers associated with stem cells and/or that have CSCs, and more especially preferably, the method for treating breast cancer as described in this patent application.

Another aspect of this invention refers to a process for obtaining a compound of formula **I** as previously defined that comprises:
(a) making a compound of formula **II** cyclic in the presence of an acid 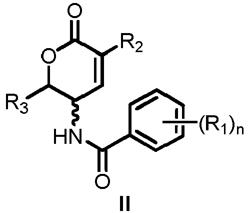 where R₁, R₂, R₃ and n have the previously described meanings; or
(b) transforming, in one or several stages, a compound of formula I into another compound of formula **I.**

Throughout this invention, the term alkyl C₁₋₁₂, as a group or part of a group, refers to a linear or branched alkyl chain that contains from 1 to 12 atoms of carbon. Examples include, methyl, ethyl, propyl, isopropyl, butyl, isobutyl, sec-butyl, ter-butyl, pentyl, isopentyl, neopentyl, hexyl, heptyl, octyl, nonyl and decanyl groups.

The alkyl C₁₋₄ group, as a group or a part of a group, refers to a linear or branched alkyl chain containing from 1 to 4 carbon atoms and includes the methyl, ethyl, propyl, isopropyl, butyl, isobutyl, sec-butyl and ter-butyl groups.

The alkenyl C₂₋₁₂ group, as a group or part of a group, refers to a linear or branched alkyl chain containing from 2 to 12 C atoms and that also contains from 1 to 6 double bonds. The alkenyl C₂₋₄ group, as a group or part of a group, refers to a linear or branched alkyl chain containing from 2 to 4 carbon atoms that additionally contains one or two double bonds. Examples include the ethenyl, 1-propenyl, 2-propenyl, isopropenyl, 1-butenyl, 2-butenyl, 3-butenyl and 1,3-butadienyl groups.

The alkynyl C₂₋₁₂ group, as a group or part of a group, refers to a linear or branched alkyl chain containing from 2 to 12 carbon atoms that additionally contains from 1 to 6 triple bonds. The alkynyl C₂₋₄ group, as a group or part of a group, refers to a linear or branched alkyl chain containing from 2 to 4 carbon atoms that additionally contains one or two triple bonds. Examples include the ethynyl, 1-propynyl, 2-propynyl, 1-butynyl, 2-butynyl, 3-butynyl and 1-3 butadiynyl groups.

The alkyl Cy₁-C₁₋₄ group refers to the group resulting from the substitution of one atom of hydrogen of the alkyl C₁₋₄ group by one Cy₁ group.

The alkenyl Cy₁-C₂₋₄ group refers to the group resulting from the substitution of one atom of hydrogen of the alkenyl C₂₋₄ group by the Cy₁ group.

The alkynyl Cy₁-C₂₋₄ group refers to the group resulting from the substitution of one atom of hydrogen of the alkynyl C₂₋₄ group by the Cy₁ group. A halogen radical or its abbreviation means fluoro, chloro, bromo or iodo.

In a compound of formula **I,** Cy₁ represents a phenyl or a saturated, partially unsaturated or aromatic 5 or 6 member heterocyclic ring that is optionally fused to a carbocyclic or heterocyclic 5 or 6 member ring that may be saturated, partially unsaturated or aromatic. Cy₁ is bound through an available C or N atom to the alkyl C₁₋₄ group. The 5 or 6 member heterocyclic ring may contain from 1 to 4 heteroatoms selected from N, O and S and where one or more available C or N atoms in Cy₁ may be optionally oxidised forming CO, SO or SO₂ groups. Cy₁ may be optionally substituted by one or more R₇ groups as indicated above in the definition of a compound of formula **I**. If they are present, the substituents of Cy₁ may be located in any of the available positions of Cy₁. Examples of Cy₁ include, phenyl, thiophyenyl, furyl, pyrrolyl, thiazolyl, isothiazolyl, oxazolyl, imadazolyl, pyrazolyl, 1,2,3-triazolyl, 1,2,4-triazolyl, tetrazolyl, 1,3,4-oxadiazolyl, 1,3,4-thiadiazolyl, 1,2,4-oxadiazolyl, 1,2,4-thiodiazolyl, pyridyl, pyrazinyl, pyrimidinyl, pyridazinyl, furanyl and indolyl.

When in the definition of Cy₁ used throughout this description specified examples refer to a ring radical in general terms, for example, pyridyl or thienyl, all the positions of possible bonds are included. Thus, for example, in the definition of Cy₁, that does not include any limitation regarding the binding position, the term pyridyl includes 2-pyridyl, 3-pyridyl and 4-pyridyl; and thiophenyl includes 2-thiophenyl and 3-thiophenyl.

When a non-aromatic ring is present as a substituent of a non-aromatic ring, this can be replaced by a hydrogen atom, or can be replaced by two hydrogen atoms on the same carbon atom thereby forming a spirane-type ring. Similarly, when a non-aromatic ring is present as a substituent of an alkyl or alkenyl group, it can be replaced by a hydrogen atom, or two hydrogen atoms and share a carbon atom with this alkyl or alkenyl group forming groups such as those shown below:

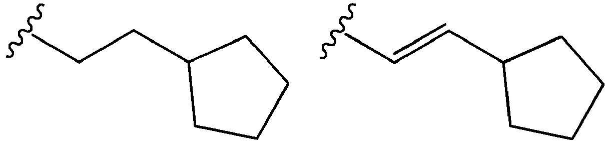

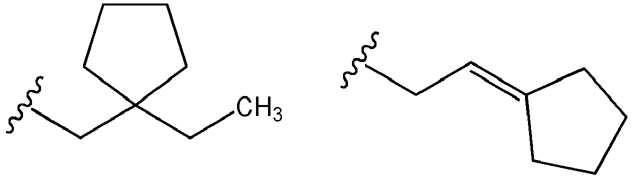

Throughout the invention, when making a reference to any given formula, the selection of a particular structural unit from the list of possible species for a specified variable is not intended to define the structural unit for the variable that appears in another location. In other words, where a variable appears more than once, the choice of species from a specified list is independent of the choice of the species for the same variable in another part of the formula (where one or more up to all the more general expressions in embodiments characterised as preferred above or below can be replaced with a more specific definition, which leads to a more preferred embodiment of the invention).

Where the plural form is used (for example, compounds, salts), this includes the singular (for example, a single compound, a single salt). "A compound" does not exclude the possibility that (for example in a pharmaceutical formulation) more than one compound of the formula **I** (or a salt thereof) is present.

Any formula given in this document is also intended to represent unlabelled forms as well as isotopically labelled forms of the compounds. Isotopically labelled compounds have structures represented by the formulas given in this document except that one or more atoms are replaced by an atom that has a higher selected atomic mass or number.

The expression "optionally substituted by one or more" means the possibility that a group may be substituted by one or more, preferably 1, 2, 3 or 4 substituents, more preferably 1, 2 or 3 substituents and still preferably by 1 or 2 substituents, provided that the group has sufficient available positions that can be substituted. If present, these substituents may be the same or different and may be located in any available position.

When in a definition of a substituent two or more groups appear with the same numbering (for example -N(alkyl C₁₋₄)₂, -NR₅R₅, -CONR₆R₆, etc.), this does not mean that they have to be identical. Each of those may be selected independently from the list of possible meanings given for this group and therefore may be the same or different.

The invention therefore refers to compounds of formula **I** as previously defined.

In another embodiment, the invention refers to a compound of formula I, where each R₁ independently represents alkyl C₁₋₁₂, alkenyl C₂₋₁₂, alkynyl C₂₋₁₂, alkyl Cy₁-C₁₋₄, alkenyl Cy₁-C₂₋₄, alkynyl Cy₁-C₂₋₄, -OR₄, -SR₄ or -NR₅R₅.

In another embodiment, the invention refers to a compound of formula I, where each R₁ independently represents alkyl C₁₋₁₂, alkyl Cy₁-C₁₋₄, -OR₄, -SR₄ or -NR₅R₅, where each alkyl C₁₋₁₂, alkenyl C₂₋₁₂, alkynyl C₂₋₁₂, alkyl Cy₁-C₁₋₄, alkenyl Cy₁-C₂₋₄ and alkynyl Cy₁-C₂₋₄ are independently and optionally substituted by one or more halogen, -O(alkyl C₁₋₄), -S(alkyl C₁₋₄), -NH(alkyl C₁₋₄) or -N(alkyl C₁₋₄)₂ groups, preferably -OR₄, -SR₄ or -NR₅R₅ groups, and still more preferably by -OR₄.

In another embodiment, the invention refers to a compound of formula **I,** where each R₁ independently represents alkyl C₁₋₁₂, alkyl Cy₁-C₁₋₄, -OR₄, -SR₄ or -NR₅R₅, preferably -OR₄, -SR₄ or -NR₅R₅, and still more preferably -OR₄.

In another embodiment, the invention refers to a compound of formula **I** where R₂ represents hydrogen or alkyl C₁₋₄, preferably hydrogen or methyl, and still more preferably hydrogen.

In another embodiment, the invention refers to a compound of formula **I** where R₃ represents hydrogen or methyl, and preferably hydrogen.

In another embodiment, the invention refers to a compound of formula **I** where n represents from 0 to 2, and still more preferably 1 or 2.

In another embodiment, the invention refers to a compound of formula **I** where each R₄ independently represents hydrogen, alkyl C₁₋₁₂ or alkyl Cy₁-C₁₋₄.

In another embodiment, the invention refers to a compound of formula **I** where:
each R₅ independently represents hydrogen or alkyl C₁₋₄; or
two R₅ groups may be bound on the same N atom forming a 5 or 6 member ring with the N atom, which may additionally contain one or two heteroatoms selected from N, S and O and are optionally substituted by one or more alkyl C₁₋₄ groups.

In another embodiment, the invention refers to a compound of formula **I** where each R₅ independently represents hydrogen or alkyl C₁₋₄.

In another embodiment, the invention refers to a compound of formula **I** where each R₆ independently represents hydrogen, alkyl C₁₋₁₂ or alkyl Cy₁-C₁₋₄, and preferably hydrogen or alkyl C₁₋₁₂, and still more preferably hydrogen or methyl.

In another embodiment, the invention refers to a compound of formula I where Cy₁ represents phenyl or a saturated, partially unsaturated or aromatic 5 or 6 member heterocyclic ring bound to the rest of the molecule through any available C or N atom, where Cy₁ may contain 1 or 2 heteroatoms in total selected from N, O and S, and where Cy₁ is optionally substituted by one or more R₇, preferably where Cy₁ is optionally substituted by one or two R₇.

In another embodiment, the invention refers to a compound of formula I where Cy₁ represents phenyl optionally substituted by one or more R₇, preferably where Cy₁ represents phenyl optionally substituted by one or two R₇, and more preferably where Cy₁ represents phenyl.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and still more preferably -OR₄; and
n represents from 0 to 2, and more preferably 1 or 2.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen; and
n represents from 0 to 2, and more preferably 1 or 2.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₃ represents hydrogen or alkyl C₁₋₄, preferably hydrogen or methyl, and more preferably hydrogen; and
n represents from 0 to 2, and more preferably 1 or 2.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
R₃ represents hydrogen or alkyl C₁₋₄, preferably hydrogen or methyl, and more preferably hydrogen; and
n represents from 0 to 2, and more preferably 1 or 2.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
n represents from 0 to 2, and more preferably 1 or 2; and
each R₄ independently represents hydrogen, alkyl C₁₋₁₂ or alkyl Cy₁-C₁₋₄.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
n represents from 0 to 2, and more preferably 1 or 2; and
Cy₁ represents phenyl or a saturated, partially unsaturated or aromatic 5 or 6 member heterocyclic ring bound to the rest of the molecule through any available C or N atom, where Cy₁ may contain 1 or 2 heteroatoms in total selected from N, O and S, and where Cy₁ is optionally substituted by one or more R₇, preferably where C_{y}1 is optionally substituted by one or two R₇.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
n represents from 0 to 2, and more preferably 1 or 2; and
Cy₁ represents phenyl optionally substituted by one or more R₇, preferably where Cy₁ represents phenyl optionally substituted by one or two R₇, and more preferably where Cy₁ represents phenyl.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
n represents from 0 to 2, and more preferably 1 or 2;
each R₄ independently represents hydrogen, alkyl C₁₋₁₂ or alkyl Cy₁-C₁₋₄; and
Cy₁ represents phenyl or a saturated, partially unsaturated or aromatic 5 or 6 member heterocyclic ring bound to the rest of the molecule through any available C or N atom, where Cy₁ may contain 1 or 2 heteroatoms in total selected from N, O and S, and where Cy₁ is optionally substituted by one or more R₇, preferably where Cy₁ is optionally substituted by one or two R₇.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
n represents from 0 to 2, and more preferably 1 or 2;
each R₄ independently represents hydrogen, alkyl C₁₋₁₂ or alkyl Cy₁-C₁₋₄; and Cy₁ represents phenyl optionally substituted by one or more R₇, preferably where Cy₁ represents phenyl optionally substituted by one or two R₇, and more preferably where Cy₁ represents phenyl.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
n represents from 0 to 2, and more preferably 1 or 2; and
each R₄ independently represents hydrogen, alkyl C₁₋₁₂ or alkyl Cy₁-C₁₋₄.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
n represents from 0 to 2, and more preferably 1 or 2; and
Cy₁ represents phenyl or a saturated, partially unsaturated or aromatic 5 or 6 member heterocyclic ring bound to the rest of the molecule through any available C or N atom, where Cy₁ may contain 1 or 2 heteroatoms in total selected from N, O and S, and where Cy₁ is optionally substituted by one or more R₇, preferably where Cy₁ is optionally substituted by one or two R₇.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
n represents from 0 to 2, and more preferably 1 or 2; and
Cy₁ represents phenyl optionally substituted by one or more R₇, preferably where Cy₁ represents phenyl optionally substituted by one or two R₇, and more preferably where Cy₁ represents phenyl.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
n represents from 0 to 2, and more preferably 1 or 2;
Cy₁ represents phenyl or a saturated, partially unsaturated or aromatic 5 or 6 member heterocyclic ring bound to the rest of the molecule through any available C or N atom, where Cy₁ may contain 1 or 2 heteroatoms in total selected from N, O and S, and where Cy₁ is optionally substituted by one or more R₇, preferably where Cy₁ is optionally substituted by one or two R₇; and
each R₄ independently represents hydrogen, alkyl C₁₋₁₂ or alkyl Cy₁-C₁₋₄.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
n represents from 0 to 2, and more preferably 1 or 2;
Cy₁ represents phenyl optionally substituted by one or more R₇, preferably where Cy₁ represents phenyl optionally substituted by one or two R₇, and more preferably where Cy₁ represents phenyl; and
each R₄ independently represents hydrogen, alkyl C₁₋₁₂ or alkyl Cy₁-C₁₋₄.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₃ represents hydrogen or alkyl C₁₋₄, preferably hydrogen or methyl, and more preferably hydrogen;
n represents from 0 to 2, and more preferably 1 or 2; and
each R₄ independently represents hydrogen, alkyl C₁₋₁₂ or alkyl Cy₁-C₁₋₄.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₃ represents hydrogen or alkyl C₁₋₄, preferably hydrogen or methyl, and more preferably hydrogen;
n represents from 0 to 2, and more preferably 1 or 2;
each R₄ independently represents hydrogen, alkyl C₁₋₁₂ or alkyl Cy₁-C₁₋₄; and
Cy₁ represents phenyl or a saturated, partially unsaturated or aromatic 5 or 6 member heterocyclic ring bound to the rest of the molecule through any available C or N atom, where Cy₁ may contain 1 or 2 heteroatoms in total selected from N, O and S, and where Cy₁ is optionally substituted by one or more R₇, preferably where Cy₁ is optionally substituted by one or two R₇.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₃ represents hydrogen or alkyl C₁₋₄, preferably hydrogen or methyl, and more preferably hydrogen;
n represents from 0 to 2, and more preferably 1 or 2;
each R₄ independently represents hydrogen, alkyl C₁₋₁₂ or alkyl Cy₁-C₁₋₄; and Cy₁ represents phenyl optionally substituted by one or more R₇, preferably where Cy₁ represents phenyl optionally substituted by one or two R₇, and more preferably where Cy₁ represents phenyl.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
R₃ represents hydrogen or alkyl C₁₋₄, preferably hydrogen or methyl, and more preferably hydrogen; and
n represents from 0 to 2, and more preferably 1 or 2.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
R₃ represents hydrogen or alkyl C₁₋₄, preferably hydrogen or methyl, and more preferably hydrogen;
n represents from 0 to 2, and more preferably 1 or 2; and
each R₄ independently represents hydrogen, alkyl C₁₋₁₂ or alkyl Cy₁-C₁₋₄.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
R₃ represents hydrogen or alkyl C₁₋₄, preferably hydrogen or methyl, and more preferably hydrogen;
n represents from 0 to 2, and more preferably 1 or 2; and
Cy₁ represents phenyl or a saturated, partially unsaturated or aromatic 5 or 6 member heterocyclic ring bound to the rest of the molecule through any available C or N atom, where Cy₁ may contain 1 or 2 heteroatoms in total selected from N, O and S, and where Cy₁ is optionally substituted by one or more R₇, preferably where Cy₁ is optionally substituted by one or two R₇.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
R₃ represents hydrogen or alkyl C₁₋₄, preferably hydrogen or methyl, and more preferably hydrogen;
n represents from 0 to 2, and more preferably 1 or 2; and
Cy₁ represents phenyl optionally substituted by one or more R₇, preferably where Cy₁ represents phenyl optionally substituted by one or two R₇, and more preferably where Cy₁ represents phenyl.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
R₃ represents hydrogen or alkyl C₁₋₄, preferably hydrogen or methyl, and more preferably hydrogen;
n represents from 0 to 2, and more preferably 1 or 2;
each R₄ independently represents hydrogen, alkyl C₁₋₁₂ or alkyl Cy₁-C₁₋₄; and
Cy₁ represents phenyl or a saturated, partially unsaturated or aromatic 5 or 6 member heterocyclic ring bound to the rest of the molecule through any available C or N atom, where Cy₁ may contain 1 or 2 heteroatoms in total selected from N, O and S, and where Cy₁ is optionally substituted by one or more R₇, preferably where Cy₁ is optionally substituted by one or two R₇.

In another embodiment, the invention refers to a compound of formula I where:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and more preferably -OR₄;
R₂ represents hydrogen or alkyl C₁₋₄, preferably hydrogen or methyl, and more preferably hydrogen;
R₃ represents hydrogen or alkyl C₁₋₄, preferably hydrogen or methyl, and more preferably hydrogen;
n represents from 0 to 2, and more preferably 1 or 2;
each R₄ independently represents hydrogen, alkyl C₁₋₁₂ or alkyl Cy₁-C₁₋₄; and Cy₁ represents phenyl optionally substituted by one or more R₇, preferably where Cy₁ represents phenyl optionally substituted by one or two R₇, and more preferably where Cy₁ represents phenyl.

In another embodiment, the invention refers to the use of the compound of formula I or a pharmaceutically acceptable salt thereof for preparing a medicine for the treatment of a disease involving cells that express at least one of the following markers: HIF-1α, HIF-2α, SOX-2, NR2F2, JAG1, PTEN, OCT4, NANOG, Klf-LF4, Notch, cMyc, miR21, miR205 or miR302; preferably for the treatment of a disease that involves the modulation and/or inhibition of HIF-2α and/or SOX-2.

In another embodiment, the invention refers to the use of the compound of formula I, or a pharmaceutically acceptable salt thereof, as described in this patent application, for preparing a medicine for the treatment of cancer and particularly cancers that express biomarkers associated with stem cells and/or have CSCs.

In another embodiment, the invention refers to a compound of formula I or a pharmaceutically acceptable salt thereof as described in this patent application, to prepare a medicine for the treatment of cancer, still more preferably of breast cancer, brain cancer, head cancer, neck cancer, liver cancer, cervical cancer, colorectal cancer, endometrial cancer, gastric cancer, lung cancer, pancreatic cancer, ovarian cancer, prostate cancer or renal cancer.

In another embodiment, the invention refers to a compound of formula I or a pharmaceutically acceptable salt thereof as described in this patent application, to prepare a medicine for the treatment and/or prevention of cancer metastasis and recurrences caused by CSCs.

In another embodiment, the invention refers to a compound of formula **I** selected from:
(3a*R*,7a*S*)-2-(3-methoxyphenyl)-7,7a-dihydro-3a*H*-pyrano[3,4-*d*]oxazole-6(4*H*)-one;
(3a*R*,7a*S*)-2-(3,5-dimethoxyphenyl)-7,7a-dihydro-3a*H*-pyrano[3,4-*d*]oxazole-6(4*H*)-one;
(3a*R*,7a*S*)-2-(3,5-bis(benzyloxy)phenyl)-7,7a-dihydro-3a*H*-pyrano[3,4-d]oxazole-6(4*H*)-one;
(3a*R*,7a*S*)-2-(3-propoxyphenyl)-7,7a-dihydro-3a*H*-pyrano[3,4-d]oxazole-6(4*H*)-one;
(3a*R*,7a*S*)-2-(3-pentyloxyphenyl)-7,7a-dihydro-3a*H*-pyrano[3,4-d]oxazole-6(4*H*)-one; and
(3a*R*,7a*S*)-2-(3-(2-methylethoxy)phenyl)-7,7a-dihydro-3a*H*-pyrano[3,4-d]oxazole-6(4*H*)-one, or
a salt thereof.

The salts of the compounds of formula **I** are preferably pharmaceutically acceptable; such salts are known in the field. In this patent application, "pharmaceutically acceptable salt" is understood to be any salt of the compound of formula **I** that can be used in contact with human and small mammal tissues without showing significant toxicity, irritation, allergic response or similar, with a reasonable benefit/risk ratio and effective for the intended use when the salt is used in a pharmaceutical composition. The compounds of this invention are able to react, for example, with a number of inorganic and organic acids to form pharmaceutically acceptable acid or base addition salts. These pharmaceutically acceptable salts and the common methods for preparing them are well known in the art. See, for example, P. Stahl, et al., Handbook of pharmaceutical salts: properties, selection and use, (VCHA/Wiley- VCH, 2002). Examples of pharmaceutically acceptable salts of carboxyl groups include salts of metals such as, for example, aluminium, salts of alkaline metals such as sodium or potassium, salts of alkaline earth metals such as calcium or magnesium and salts of ammonium or substituted ammonium, for example, those with (lower)alkyl-amines such as triethylamine, hydroxyalkylamines such as 2-hydroxyethylamine, bis-(2-hydroxyethyl)-amine or tris-(2-hydroxyethyl)-amine, cycloalkylamines such as bicyclohexylamine, or with procaine, dibenzylpiperidine, N-benzyl-b-phenethylamine, dehydroabiethylamine, N,N'-bisdehydroabiethylamine, glucamine, N-methylglucamine or pyridine-type bases such as pyridine, collidine or quinoline.

Thus the compounds of this invention containing one or more basic nitrogen atoms may form salts with acids, either organic or inorganic. Examples of such salts include: salts with inorganic acids such as hydrochloric acid, hydrobromic acid, hydroiodic acid, nitric acid, perchloric acid, sulphuric acid or phosphoric acid; and salts with organic acids such as methanesulfonic acid, trifluoromethanesulfonic acid, ethanesulfonic acid, benzenesulfonic acid, p-toluenesulfonic acid, fumaric acid, oxalic acid, acetic acid, maleic acid, ascorbic acid, citric acid, lactic acid, tartaric acid, malonic acid, glycolic acid, succinic acid and propionic acid. Some compounds of this invention may contain one or more acid protons and therefore may also form salts with bases. Examples of such salts include: salts with inorganic cations such as sodium, potassium, calcium, magnesium, lithium, aluminium, zinc, etc.; and salts formed with pharmaceutically acceptable amines such as ammonium, alkylamines, hydroxyalkylamines, lysine, arginine, *N*-methylglucamine, procaine and similar.

There is no limit to the type of salt that can be used, with the condition that when used for therapeutic purposes they must be pharmaceutically acceptable. Pharmaceutically acceptable salts are understood to be salts that, in medical judgement, are suitable for use in contact with the tissues of human beings and other mammals without causing undue toxicity, irritation, allergic response or similar. Pharmaceutically acceptable salts are widely known by any expert in the field.

The salts of a compound of formula **I** can be obtained during final isolation and purification of the compounds of the invention or may be prepared by treating a compound of formula **I** with a sufficient amount of desired acid or base to yield a salt in a conventional way. The salts of compounds of formula **I** can in turn be transformed into other salts of compounds of formula **I** by ion exchange using an ion exchange resin.

The compounds of formula **I** and their salts can differ in certain physical properties, but they are equivalent for the purposes of the invention. All the salts of compound of formula **I** are included in the scope of the invention.

The compounds of this invention may form complexes with solvents in which they are made to react or from which they can be precipitated or crystallised. These complexes are known as solvates. As used here, the term solvate refers to a complex of variable stoichiometry formed by a solute (a compound of formula **I** or a salt thereof) and a solvent. Examples of solvents include pharmaceutically acceptable solvents such as water, ethanol and similar. A complex with water is known as a hydrate. The solvates of the compounds of the invention (or their salts), including hydrates, are included in the scope of the invention.

The compounds of formula **I** may exist in different physical forms, that is in amorphous or crystalline form. Thus, compounds of this invention may have the ability to crystallise in more than one form, a characteristic known as polymorphism. Polymorphs can differ in various physical properties well known by experts in the field such as, for example, their X-ray diffraction spectra, melting points or solubility. All the physical forms of compound of formula **I**, including all the polymorphic forms ("polymorphs"), are included in the scope of this invention.

Some compounds of this invention may exist in the form of various diastereoisomers and/or various optical isomers. Diastereoisomers can be separated by conventional techniques such as chromatography or fractional crystallization. Optical isomers can be resolved using conventional techniques of optical resolution, to give optically pure isomers. This resolution can be performed on the synthesis intermediates that are chiral or on the products of formula **I**. The optically pure isomers can also be obtained individually using enantiospecific synthesis. This invention covers both individual isomers and their mixtures (for example racemic mixtures or diastereoisomers), whether they are obtained by synthesis or by physical mixing.

The compound of formula **I** can be obtained by following the processes described below. As will be obvious to an expert in the field, the precise method used for the preparation of a given compound may vary depending on its chemical structure. Also, in some of the processes detailed below, it may be necessary or useful to protect active or labile groups using conventional protective groups. Both the nature of these protective groups and the processes for introducing and removing them are well known and form part of the state of the art (see for example, Greene T.W. and Wuts P.G.M, "Protective Groups in Organic Synthesis", John Wiley & Sons, 4a edition, 2006). By way of example, the tetrahydropyranyl group can be used as a protective group for the amino function. When there is a protecting group, a subsequent de-protection stage will need to be performed under the normal conditions of organic synthesis, as described in the reference above.

Except when otherwise indicated, in the methods described below the meanings of the various substituents have the meanings described above in relation to a compound of the formula **I**.

Thus, compounds of formula **I** as described in this patent application can be obtained by processes known by an expert in the field, in particular by the processes described in the examples.

Some compounds of this invention can also be obtained from other compounds of formula **I** via transformation reactions of suitable functional groups in one or more stages using reactions that are widely known in organic chemistry under common experimental conditions.

These transformations can be carried out on the R₁ or R₂ groups and may include for example:
substitution of a primary or secondary amine by treatment with an alkylating agent in standard conditions, or by reductive amination, that is, by treatment with an aldehyde or ketone in the presence of a reducing agent such as sodium cyanoborohydride or sodium triacetoxyborohydride;
transformation of an amine into a sulphonamide by reaction with a sulfonyl halide, such as sulfonyl chloride, optionally in the presence of catalytic amounts of a base such as 4-dimethylaminopyridine in a suitable solvent such as dioxane, chloroform, dichloromethane or pyridine, optionally in the presence of a base such as triethylamine or pyridine;
alkylation of an amide by treatment with an alkylating agent in basic conditions; conversion of an alcohol into an ether, ester or carbamate under standard conditions;
alkylation of a thiol to obtain a thioether under standard conditions;
partial or total oxidation of an alcohol to obtain ketones, aldehydes or carboxylic acids in standard oxidation conditions;
reduction of an aldehyde or ketone to an alcohol by treatment with a reducing agent such as sodium borohydride;
reduction of a carboxylic acid or a derivative of a carboxylic acid to an alcohol by treatment with a reducing agent such as diisobutylaluminum hydride or LiAlH₄;
reduction of an amide to an amine by treatment with a reducing agent such as LiAlH₄;
oxidation of a thioether to a sulphoxide or sulphone under standard conditions; transformation of an alcohol into a halide by treatment with SOCl₂, PBr₃, tetrabutylammonium bromide in the presence of P₂O₅, or PI₃;
transformation of a halide into a alkylsulfone or arylsulfone by treatment with sodium alkyl sulfonate or sodium aryl sulfonate respectively; and transformation of a halogen atom into an amine by reaction with an amine, optionally in the presence of a suitable solvent and preferably by heating it.

Similarly, any of the aromatic rings of the compounds of this invention can undergo aromatic electrophilic substitution or aromatic nucleophilic substitution reactions, widely described in the bibliography.

Some of these interconversion reactions are explained in more detail in the examples.

As will be obvious to experts in the field, these interconversion reactions can be carried out both on compounds of formula **I** and on any of their suitable synthesis intermediates.

A previously mentioned, the invention refers to the use of a compound of formula **I** or a pharmaceutically acceptable salt thereof for making a medicine for the treatment of a disease involving cells expressing at least one of the following markers: HIF-1α, HIF-2α, SOX-2, NR2F2, JAG1, PTEN, OCT4, NANOG, Klf-LF4, Notch, cMyc, miR21, miR205 or miR302; preferably for the treatment of a disease that involves modulation and/or inhibition of HIF-2α and/or SOX-2 such as, for example but without limitation, cancer. More preferably, a method for the treatment of cancer and preferably cancers expressing biomarkers associated with stem cells and/or that have CSCs, and more especially preferably, the method for treating breast cancer as described in this patent application.

In this invention, the term "cancers expressing biomarkers associated with stem cells" refers to the presence in the cancers of cells that present markers associated with stem or progenitor cells. The detection of these markers in a cancer can be linked to the presence of cells with a "pro-tumour" phenotype or CSCs, or to an incomplete transformation of this phenotype.

In this invention, the term "cancer stem cells" (CSCs), also known as "tumour initiating cells" (TICs) refers to a subtype of cancer cells with the phenotypic characteristics associated with non-tumour embryonic stem cells, specifically the ability to give rise to a heterogeneity of the cell types found in a tissue such as a solid tumour. CSCs have diverse phenotypic characteristics but share the presence of markers associated with embryonic stem and progenitor cells. CSCs are tumourigenic (tumour formers), compared to other tumour cells that do not have the potential of originating tumours but only of increasing the tumour load by uncontrolled proliferation. The CSCs markers include: CD44, CD24, CD133, ALDH1, CD90, EpCAM, ABCG5, CD34, see Table 1.

**Table 1: CSC markers known in the state of the art.**

| **Tumour type** | **Phenotype of CSC markers** |
|---|---|
| Leukaemia | CD34⁺CD38⁻HLA-DR-CD71⁻CD90⁻CD11⁻CD123⁺ |
| Breast cancer | ESA⁺CD44⁺CD24^{-/low}Lineage⁻, ALDH-1^{high} |
| Liver cancer | CD133⁺, CD49f⁺, CD90⁺ |
| Brain cancer | CD133⁺, BCRP1⁺, A2B5⁺, SSEA-1⁺ |
| Lung cancer | CD133⁺, ABCG2^{high} |
| Colon cancer | CD133⁺, CD44⁺, CD166⁺, EpCAM⁺, CD24⁺ |
| Multiple myeloma | CD138- |
| Prostate cancer | CD44⁺, a2β1^{high}, CD133⁺ |
| Pancreatic cancer | CD133⁺, CD44⁺, EpCAM⁺, CD24⁺ |
| Melanoma | CD20⁺ |
| Throat cancer | CD44⁺ |
| Glioblastoma | CD133⁺ |
| Ovarian cancer | SP⁺ |
| Gastric cancer | CD44⁺, SP⁺ |
| Head and neck squamous cell carcinomas (HNSCC) | SP⁺, CD44⁺ |
| Osteosarcoma | CD133⁺, CD117⁺, Stro-1⁺,SP⁺, ALDH⁺ |
| Chondrosarcoma | CD133⁺, SP⁺ |
| Sinovial sarcoma | CD133⁺ |
| Ewing's sarcoma | CD133⁺, ALDH⁺ |
| Rhabdomyosarcoma | CD133⁺ |
| Mesenchymal neoplasms | SP⁺ |

Throughout this description, the term "treatment" refers to eliminating, reducing or lessening the cause or effects of a disease. For the purposes of this invention, treatment includes, without limitation, alleviating, reducing or removing one or more of the symptoms of the disease; reducing the degree of the disease, stabilising (i.e. not worsening) the status of the disease, delaying or slowing down the progression of the disease, alleviating or improving the status of the disease and reaching remission (either total or partial).

In this invention, the term "treatment of a disease that presents CSCs" refers to the disease in which all or some of the cells involved in the aetiology of the disease express at least one marker for stem cells such as, for example but without limitation, those appearing in Table 1. Preferably, given the tumourigenic character of CSCs, reference is made to tumour processes or cancer.

In another embodiment, the invention refers to the use of the compound of formula **I** or a pharmaceutically acceptable salt thereof to prepare a medicine for the treatment of a disease that involves the treatment of a disease involving cells expressing at least one of the following markers: HIF-1α, HIF-2α, SOX-2, NR2F2, JAG1, PTEN, OCT4, NANOG, Klf-LF4, Notch, cMyc, miR21, miR205 or miR302.

In the present invention, the term "expression of HIFs" is used as the expression of any of the known "hypoxia-inducible factors" (HIFs) and/or their target genes. The HIFs are transcription factors that respond to changes in the cellular environment, specifically to changes in the level of oxygen. HIF factors consist of three subunits, alpha (A, α) and beta (B, β); the various genes that form part of the family are: HIF-1α (Gene ID: 3091, HIF1A hypoxia-inducible factor 1 alpha subunit), HIF-1β (Gene ID: 405, gen ARNT, aryl hydrocarbon receptor nuclear translocator), HIF-2α (Gene ID: 2034, EPAS1 gene, endothelial PAS domain protein 1), HIF-2β (Gene ID: 9915, ARNT2 gene, aryl-hydrocarbon receptor nuclear translocator 2), HIF-3α (Gene ID: 64344, HIF3A gene, hypoxia inducible factor 3, alpha subunit) and HIF-3β (Gene ID: 406, ARNTL gene, aryl-hydrocarbon receptor nuclear translocator 3). Specifically, in this invention, the HIF inhibitors are potential inhibitors of the HIF-α subunits.

In this invention, the term "SOX-2 expression" (Gene ID: 6657; SRY (sex determining region Y)-box 2) refers to a transcription factor regulated by HIF-2α, which is essential for maintaining self-renewal, or pluripotency, of undifferentiated embryonic stem cells. SOX-2 is a member of the SOX family of transcription factors, which have been demonstrated to play a key role in various early stages of mammalian development.

In the present invention, the term "NR2F2 expression" (Gene ID: 7026; nuclear receptor subfamily 2, group F, member 2) refers to a nuclear receptor of the superfamily of thyroid steroid hormones. This protein is a transcription factor involved in the regulation of multiple genes. There are many variants that come from alternative splicing.

In this invention, the term "JAG1 expression" (Gene ID: 182) refers to a protein that interacts with Notch receptors and is involved in its signalling pathway. It has been related to haematopoiesis and in early and late cardiovascular development.

In this invention, the term "PTEN expression" (Gene ID: 5728; Phosphatase and tensin homolog) refers to the expression of the phosphatidylinositol-3,4,5-trisphosphate 3-phosphatase enzyme (EC 3.1.3.67) that catalyses the hydrolysis of the phosphate group of carbon 3 of phosphatidilinositol-3,4,5-triphosphate. PTEN is a tumour suppressor as it acts as a phosphatase protein of double specificity on the tyrosine, serine and threonine residues of phosphorylated proteins. It antagonises the PI3K-AKT/PKB signalling pathway, dephosphorylating the phosphoinositides and therefore modulating the progression of the cell cycle and cell survival.

In this invention, the term "OCT4 expression" refers to a transcription factor critically involved in undifferentiated embryonic stem cell self-renewal. OCT4 (Gene ID: 5460; octamer-binding transcription factor 4) is also known as POU5F1 (POU class 5 homeobox 1), OCT3, OCT4, OTF3, OTF4, OTF-3, Oct-3, Oct-4. The expression of this protein is also regulated in hypoxia by HIF-2α, and as such is frequently used as a marker for discriminating undifferentiated cells.

In this invention, the term "NANOG expression" refers to a transcription factor critically involved in undifferentiated embryonic stem cell self-renewal. In this invention, references to NANOG also refer to the transcription factor coded by the Nanog gene (Gene ID: 79923) and the NanoGP8 gene (Gene ID: 388112), a pseudogene of the NANOG transcription factor.

In this invention, the term "Klf-LF4 expression" (Gene ID: 9314, KLF4, factor 4 Kruppel-like) refers to a transcription factor coded in humans by the klf4 gene. In embryonic cells KLF4 has been demonstrated to be a good indicator of the plasticity of stem cells to dedifferentiate.

In this invention, the term "cMYC expression" refers to the Myc genes, a family of proto-oncogenes comprising several members (L-myc, N-myc and c-myc) that code for proteins of the cell nucleus that bind to DNA and facilitate its transcription, thereby regulating the activity of other genes. There is a great deal of scientific literature that illustrates the interaction of HIFs and MYC in the processes of development of cancer disease as factors indicating poor prognosis.

Throughout the description, the term "miR21 expression" (Gene ID: 406991) refers to a microRNA with tumour suppressant activity that is linked to a large variety of cancers such as breast, ovarian, cervical, colon, lung, liver, brain, oesophagus, prostate, pancreatic and thyroid cancers.

Throughout the description, the term "miR205 expression" (Gene ID: 406998) refers to a microRNA that is frequently silenced in advanced cancers. It is involved in the epithelial-mesenchymal transition and tumour invasion. Throughout the description, the term "miR302 expression" (Gene ID: 407028) refers to a microRNA with regulatory activity on OCT4, present in pluripotent cells.

In another embodiment, the invention refers to the use of the compound of formula **I** or a pharmaceutically acceptable salt thereof, as described in this patent application, to prepare a medicine for the treatment of a disease involving cells that express at least one of the following markers: HIF-1α, HIF-2α, SOX-2, NR2F2, JAG1, PTEN, OCT4, NANOG, Klf-LF4, Notch, cMyc, miR21, miR205 or miR302; preferably for the treatment of a disease involving the modulation and/or inhibition of HIF-2α and/or SOX-2 such as, for example but without limitation, cancer. More preferably, use of the compound of formula I for the treatment of cancer and, preferably of cancers that express biomarkers associated with stem cells and/or present CSCs.

In another embodiment, the invention refers to the use of the compound of formula **I** or a pharmaceutically acceptable salt thereof as described in this patent application to prepare a medicine for the treatment of cancer, more preferably of breast cancer, brain cancer, head cancer, neck cancer, liver cancer, cervical cancer, colorectal cancer, endometrial cancer, gastric cancer, lung cancer, pancreatic cancer, ovarian cancer, prostate cancer and renal cancer, more preferably for the treatment of breast and brain cancer and still more preferably for the treatment of breast cancer.

In another embodiment, the invention refers to a compound of formula **I** or a pharmaceutically acceptable salt thereof as described in this patent application to prepare a medicine for the treatment and/or prevention of cancer metastasis and recurrence caused by the presence of CSCs.

In this invention, the term "cancer" is defined as a neoplastic disease with transformation of cells, which proliferate in an abnormal and uncontrolled manner. Preferably, but without limitation, reference is made to solid tumours in: breast, brain, liver, cervix, colorectum, endometrium, stomach, lung, pancreas, ovary, prostate and kidney.

The term "metastasis" is defined as the propagation of a cancerous focus in an organ other than that in which it started and to the result of this propagation. It generally occurs via the blood or lymphatic system. Approximately 98% of deaths due to undetected cancer are due to metastasis.

The terms "cancer recurrence" or "cancer relapse" are defined as the reappearance of a neoplasm sometime after it was first suffered.

As already mentioned above, the invention also refers to a method for treating a disease involving cells that express at least one of the following markers: HIF-1α, HIF-2α, SOX-2, NR2F2, JAG1, PTEN, OCT4, NANOG, Klf-LF4, Notch, cMyc, miR21, miR205 and miRNA302; through the application of a compound of formula (I) as described in the first aspect of this invention, or a pharmacological composition as described above, the method for treatment is through the modulation and/or inhibition of HIF-2α and/or SOX-2, more preferably to a method for treatment of cancer and still more preferably to a method for the treatment of breast cancer as described in this patent application.

As mentioned above, the invention also refers to a pharmaceutically acceptable composition that comprises a compound of the invention (or a pharmaceutically acceptable salt thereof) and one or more pharmaceutically acceptable excipients. The excipients must be "acceptable" in the sense of being compatible with the other ingredients of the composition and must not be harmful to the person taking this composition.

As previously mentioned, the invention also refers to a pharmaceutically acceptable composition that comprises more than one compound of formula **I** and/or other pharmaceutical active ingredients.

These compositions can be obtained by pharmaceutical manufacturing methods widely known by the expert in the pharmaceutical sector.

The compounds of this invention can be administered in the form of any pharmaceutical formulation, the nature of which, as is well known, will depend on the nature of the active ingredient and its route of administration. In principle, any route of administration can be used, for example intravenous (IV), intramuscular (IM), subcutaneous (SC), intradermal (ID), intraperitoneal (IP), intrathecal, i.e. in the space surrounding the spinal cord (IT), intraocular (IO), intrapleural (IPL), intrauterine (IU), rectal, vaginal, topical, intratumoural and similar.

The route of administration of the active compound in the form of a pharmaceutical composition can be dependent on the problem to be solved and on the patient.

If necessary, the compound of the invention can be administered as a prodrug. The term "prodrug" indicates an agent that is converted into the original drug "in vivo". In certain situations, a prodrug can be easier to administer than the original drug. For example, the prodrug can be bioavailable for oral administration but not the original drug, or the prodrug can have better solubility that enables its intravenous administration.

The compounds of this invention can be mixed with pharmaceutically acceptable excipients that act as diluents or vehicles, compatible with the active ingredient, in amounts suitable to be used in therapeutically acceptable methods. Suitable excipients are, for example but without limitation, water, saline solution, dextrose, glycerol, ethanol or similar and combinations of these. In addition, if desired, the composition can contain substances such as wetting agents or emulsifiers, pH buffers and similar, that stabilise or improve the effectiveness of the active ingredients.

The solid compositions for oral administration include tablets, granules and capsules. In any case, the method of manufacture is based on a simple mixture, dry or wet granulation of the active ingredient with excipients. These excipients can be, for example, diluents such as lactose, microcrystalline cellulose, mannitol or calcium hydrogen phosphate; agglutinating agents such as, for example, starch, gelatin or polyvinylpyrrolidone; disintegrants such as sodium carboxymethyl starch or croscarmellose sodium; and lubricating agents such as, for example, magnesium stearate, stearic acid or talc. The tablets can also be coated with suitable excipients and by known techniques in order to delay their disaggregation and absorption in the gastrointestinal tract and thereby achieve a sustained action over a longer period of time, or simply to improve their organoleptic properties or stability. The active ingredient can also be incorporated by coating on inert pellets by the use of natural or synthetic film-forming polymers. It is also possible to make soft gelatin capsules where the active ingredient is mixed with water or oily medium, for example, coconut oil, liquid paraffin or olive oil.

It is also possible to obtain powders and granules for the preparation of oral suspensions by adding water, mixing the active ingredient with dispersing agents or wetting agents, suspending agents and preserving agents. It is also possible to add other excipients, for example, sweeteners, flavourings and colouring agents.

As liquid forms for oral administration, it is possible to include emulsions, solutions, suspensions, syrups and elixirs that contain commonly used inert diluents such as distilled water, ethanol, sorbitol, glycerol, polyethylene glycols (macrogol) and propylene glycol. These compositions can also contain stabilizers, thickening agents, excipients such as wetting agents, suspending agents, sweeteners, flavourings, preservatives and pH buffers and regulators.

Injectable preparations of this invention for parenteral administration comprise sterile solutions, suspensions or emulsions in an aqueous or nonaqueous solvent such as propylene glycol, polyethylene glycol or vegetable oils. These compositions can contain excipients such as wetting agents, emulsifiers, dispersants and preservatives. They may be sterilised by any of the known methods or prepared as sterile solid compositions that are to be dissolved in water or any other sterile injectable medium immediately before their use. It is also possible to start from sterile raw materials and maintain these conditions during the whole manufacturing process.

For rectal administration, the active ingredient can be preferably formulated as a suppository in an oily base such as, for example, plant oils or solid semi-synthetic glycerides, or in a hydrophilic base such as polyethylene glycols (macrogol).

The compounds of the invention can also be formulated for topical application for the treatment of pathologies in areas or organs accessible via this route such as eyes, skin and intestinal tract. Formulations include creams, lotions, gels, powders, solutions and patches in which the compound is dispersed or dissolved in suitable excipients.

For nasal administration or by inhalation, the compound can be formulated in the form of an aerosol from which it is released with the use of suitable propellants.

The dose and dose frequency will vary depending on the nature and severity of the disease to be treated, age, general condition and the patient's weight, as well as the specific compound administered and the route of administration, among other factors. By way of example, a suitable dose range varies between around 0.01 mg/kg and around 100 mg/kg per day, which can be administered as a single dose or in several doses.

In any case, the effective dose for the administration of the pharmaceutical composition (or inhibitors) depends on the effect of the compounds and their potency as inhibitors. The term "effective dose" refers to the amount of active compound that is sufficient to cause the desired effect in which the symptoms of the disease are attenuated. The dose used must not be so high as to cause unwanted side effects, where their clinical evaluation makes them into adverse effects that are not therapeutically treatable, hyper viscosity syndrome, pulmonary oedema, congestive heart failure and similar. Generally the dose will vary with age, condition, sex, and spread of the disease in the patient and can be determined in each case.

Throughout the description and the claims, the term "comprise" and its variants does not intend to exclude other technical characteristics, additives, components or steps. For experts in the field, other objects, advantages and characteristics of the invention will emerge partly from the description and partly from the practice of the invention.

The following examples and figures are provided for the purposes of illustration and are not intended to be limiting of this invention.

### BRIEF DESCRIPTION OF THE FIGURES

**Fig. 1****.** Shows the inhibitory activity of compound IQM11PS04 on tumour line MDA_MB-436 / HRE9X-LUC.
**Fig. 2****.** Shows the inhibitory activity of IQM11PS04 on expression of the promoting region of enhancer R1 of SOX-2 in the breast tumour line MDA_MB-436 / HRE9X-LUC.
**Fig. 3****.** Shows the activity of IQM11PS04 on the formation of mammospheres in cell lines MCF7, MDA-MB-436 and BT474.

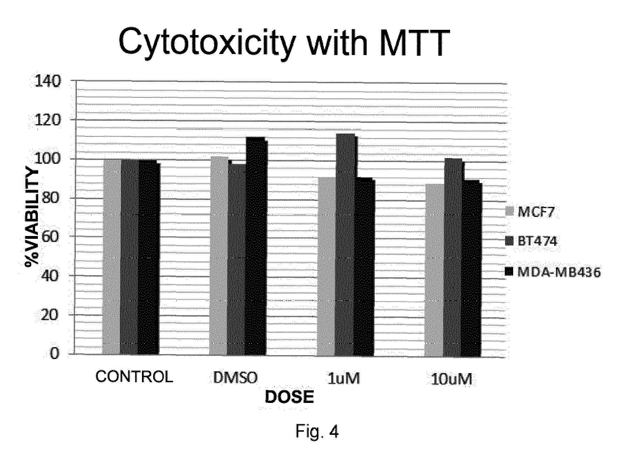

**Fig. 4****.** Shows the cytotoxicity test with MTT. The inhibitor does not affect cell viability of breast tumour lines MCF-7, BT474 and MDA-MB-436.

### EXAMPLES

### General synthesis process for compounds of formula I

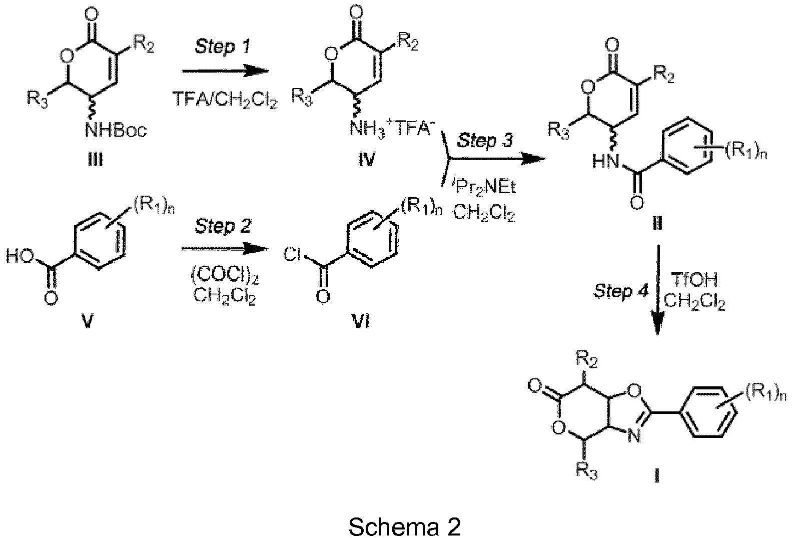

Compounds of formula **I** can be prepared following the process described in schema 2 where R₁, R₂, R₃ and n have the meanings described for a compound of formula **I:**
Step 1. A solution of the lactone **III** (1 eq) in anhydrous dichloromethane was cooled to 0 °C in an argon atmosphere and at room temperature. Trifluoroacetic acid (TFA) (21 eq) was added to the solution. The mixture was stirred at 0 °C for 5 minutes and then removed from the bath so that the mixture could slowly reach room temperature and stirring was continued for 1 hour. When the reaction was completed, checked by TLC (hexane/AcOEt - 1:3), the mixture was diluted with dichloromethane and the solvent evaporated at reduced pressure. The process of dilution and evaporation of the solvent was repeated 4 times in order to remove the remains of trifluoroacetic acid. The product **IV** obtained was used without additional purification in step 3.
Step 2. The corresponding carboxylic acid **V** (1 eq) was dissolved in anhydrous dichloromethane in an argon atmosphere and at room temperature. Dimethylformamide (catalytic amount) was added followed by a 2M oxalic chloride solution ((COCl)₂) in dichloromethane (3 eq). The mixture was stirred at room temperature for 1 hour, checking the disappearance of the acid by TLC (hexane/AcOEt - 4:1). The mixture was diluted with dichloromethane and the solvent evaporated at reduced pressure. The product **VI** obtained was used without additional purification in step 3.
Step 3. The ammonium salt of **IV** was dissolved in anhydrous dichloromethane under argon and at room temperature. Next, N,N-diisopropylethylamine (ⁱPr₂Net) (1.5eq) was added via a syringe followed by a solution of the acid chloride **VI** in anhydrous dichloromethane via a syringe under argon atmosphere and at room temperature. The mixture was stirred for 1 hour at room temperature. When the reaction was judged to be complete by TLC (hexane/AcOEt - 1:1), the mixture was diluted with dichloromethane, washed with a saturated aqueous solution of ammonium chloride, a saturated aqueous solution of sodium bicarbonate and a saturated aqueous solution of sodium chloride. The organic phase was dried with anhydrous magnesium sulphate, filtered and the solvent removed at reduced pressure. The crude product **II** was purified by chromatography on a silica column using a mixture of hexane/ethyl acetate as the eluent.
Step 4. A solution of compound **II** (1 eq), obtained in step 3, was prepared in anhydrous dichloromethane under argon and at room temperature. To this, triflic acid (TfOH) (10 eq) was added via a syringe. The mixture was stirred at room temperature for 2 hours. When the reaction was complete, monitored by TLC (hexane/AcOEt - 1:5), the mixture was diluted with dichloromethane, the organic phase was basified with a saturated aqueous solution of potassium carbonate and extracted with dichloromethane. The organic extract was dried with anhydrous magnesium sulphate, filtered and the solvent removed. The crude product obtained was purified on a silica gel column eluting a mixture of hexane/AcOEt, yielding the pure compound **I**.

### Example 1. Synthesis of 2-(3-methoxyphenyl)-7,7a-dihydro-3aH-pyrano[3,4-d]oxazole-6(4H)-one (IQM11PS01)

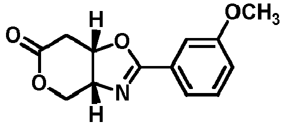

This compound was obtained using the process described using 3-methoxybenzoic acid (overall yield: 70%).

¹H NMR (400 MHz, CDCl₃) δ (ppm): 7.47 (ddd, *J* = 7.7, 1.5, 0.9 Hz, 1 H), 7.41 (dd, *J* = 2.6, 1.5 Hz, 1 H), 7.35 - 7.25 (m, 1 H), 7.03 (ddd, *J* = 8.3, 2.7, 1.0 Hz, 1 H), 5.24 (ddd, *J* = 9.9, 4.3, 2.7 Hz, 1 H), 4.66 (ddd, *J* = 10.0, 3.1, 1.9 Hz, 1 H), 4.59 (dd, *J* = 12.3, 2.0 Hz, 1 H), 4.40 (dd, *J* = 12.4, 3.2 Hz, 1 H), 3.82 (s, 3H), 3.07 (dd, *J* = 16.2, 2.8 Hz, 1 H), 2.77 (dd, *J* = 16.2, 4.2 Hz, 1 H). ¹³C NMR (100 MHz, CDCl₃) δ (ppm): 169.0, 165.2, 159.4, 129.5, 120.9, 118.8, 112.7, 75.1, 68.7, 64.1, 55.4, 34.3. HPLC-MS (ES+): Gradient MeCN/H₂O 10:90 to 100:0 (5 min), tr: 3.5 min, [M+H]⁺ = 248.9. Elemental analysis (EA): calculated for C₁₃H₁₃NO₄: C, 63.15%; H, 5.30%; N, 5.67%; found: C, 63.31%; H, 5.48%; N, 5.69%. Optical rotation: [α]_{D} = -75 (c = 1, CHCl₃).

### Example 2. Synthesis of 2-(3,5-dimethoxyphenyl)-7,7a-dihydro-3aH-pyrano[3,4-d]oxazole-6(4H)-one (IQM11PS02).

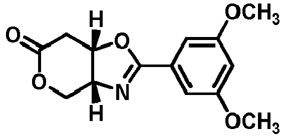

Obtained following the process described using 3,5-dimethylbenzoic acid (overall yield: 50%).

¹H NMR (400 MHz, CDCl₃) δ (ppm): 7.06 (d, *J* = 2.4 Hz, 1 H), 6.60 (t, J = 2.4 Hz, 1 H), 5.27 (ddd, *J* = 9.8, 4.1, 2.7 Hz, 1 H), 4.69 (ddd, *J* = 10.1, 2.8, 2.0 Hz, 1 H), 4.62 (dd, *J* = 12.4, 1.8 Hz, 1 H), 4.42 (dd, *J* = 12.4, 3.1 Hz, 1 H), 3.09 (dd, J = 16.2, 2.7 Hz, 1H), 2.79 (dd, *J =* 16.2, 4.2 Hz, 1 H). ¹³C NMR (100 MHz, CDCl₃) δ (ppm): 169.0, 165.1, 160.6, 160.5, 128.0, 106.0, 105.0, 75.1, 68.6, 64.1, 55.5, 34.2. HPLC-MS (ES+): Gradient MeCN/H₂O 10:90 to 100:0 (5 min), tr: 3.9 min, [M+H]⁺ = 278.1. Elemental analysis (AE): calculated for C₁₄H₁₅NO₅: C, 60.64%; H, 5.45%; N, 5.05%; found: C, 60.73%; H, 5.61%; N, 4.89%. Optical rotation: [α]_{D} = -65 (c = 1, CHCl₃).

### Example 3. Synthesis of 2-(3,5-bis(benzyloxy)phenyl)-7,7a-dihydro-3aH-pyrano[3,4-d]oxazole-6(4H)-one (IQM11PS03)

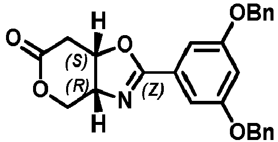

Obtained following the process described using 3,5-bis(benzyloxy)benzoic acid (overall yield: 70%).

¹H NMR (300 MHz, CDCl₃) δ (ppm): 7.50 - 7.32 (m, 10H), 7.20 (dd, *J* = 2.2, 0.7 Hz, 2H), 6.76 (t, *J* = 2.2 Hz, 1 H), 5.26 (ddd, *J* = 10.0, 4.3, 2.8 Hz, 1 H), 5.07 (s, 4H), 4.69 (dt, *J* = 10.1, 2.5 Hz, 1 H), 4.63 (dd, *J* = 12.4, 2.0 Hz, 1 H), 4.43 (dd, *J* = 12.3, 3.1 Hz, 1 H), 3.10 (dd, *J* = 16.2, 2.7 Hz, 1 H), 2.80 (dd, *J* = 16.2, 4.3 Hz, 1 H). ¹³C NMR (75 MHz, CDCl₃) δ (ppm): 168.5, 165.0, 159.9, 136.6, 128.5, 127.9, 127.4, 107.7, 106.6, 75.2, 70.4, 68.5, 64.2, 34.2. HPLC-MS (ES+): Gradient MeCN/H₂O 60:40 to 100:0 (5 min), tr: 2.5 min, [M+H]⁺ = 430.0. Elemental analysis (AE): calculated for C₂₆H₂₃NO₅: C, 72.71%; H, 5.40%; N, 3.26%; found: C, 72.48%; H, 5.57%; N, 3.23%. Optical rotation: [α]_{D} = -42 (c = 1, CHCl₃).

### Example 4. Synthesis of 2-(3-propoxyphenyl)-7,7a-dihydro-3aH-pyrano[3,4-d] oxazole-6(4H)-one (IQM11PS04)

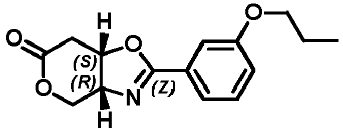

Obtained following the process described using 3-propoxybenzoic acid (overall yield: 70%).

¹H NMR (300 MHz, CDCl₃) δ (ppm): 7.47 (m, 1 H), 7.41 (m, 1 H), 7.35-7.25 (m, 1 H), 7.03 (ddd, *J* = 8.3, 2.7, 1.0 Hz, 1 H), 5.24 (ddd, *J* = 9.9, 4.3, 2.7 Hz, 1 H), 4.64 (ddd, *J* = 10.0, 3.1, 1.9 Hz, 1 H), 4.59 (dd, *J* = 12.3, 2.0 Hz, 1 H), 4.42 (dd, *J =* 12.4, 3.2 Hz, 1H), 3.96 (t, *J=* 8.0, 2H), 3.10 (dd, *J* = 16.2, 2.8 Hz, 1 H), 2.80 (dd, *J* = 16.2, 4.2 Hz, 1 H), 1.82 (m, 2H), 1.06 (t, *J* = 8.0, 3H). ¹³C NMR (75 MHz, CDCl₃) δ (ppm): 168.8, 165.4, 159.4, 129.6, 127.9, 121.0, 119.4, 114.2, 75.3, 70.0, 68.9, 64.5, 34.5, 29.8, 22.7, 10.6. HPLC-MS (ES+): Gradient MeCN/H₂O 10:90 to 100:0 (5 min), tr: 4.3 min, [M+H]⁺ = 277.3. Elemental analysis (AE): calculated for C₁₅H₁₇NO₄: C, 65.44%; H, 6.22%; N, 5.09%; found: C, 65.28%; H, 6.27%; N, 5.23%. Optical rotation: [α]_{D} = -72 (c = 1, CHCl₃).

### Example 5. Synthesis of 2-(3-pentyloxyphenyl)-7,7a-dihydro-3aH-pyrano[3,4-d]oxazole-6(4H)-one (IQM11PS05)

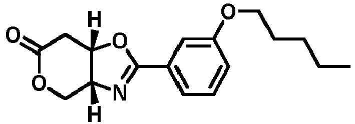

Obtained following the process described using 3-pentoxybenzoic acid (overall yield: 60%).

¹H NMR (400 MHz, CDCl₃) δ (ppm): 7.45 (dt, *J* = 7.6, 1.2 Hz, 1 H), 7.40 (dd, *J* = 2.6, 1.5 Hz, 1 H), 7.28 (dd, *J* = 8.2, 7.7 Hz, 1 H), 7.01 (ddd, *J* = 8.2, 2.7, 1.0 Hz, 1 H), 5.22 (ddd, *J =* 10.0,4.3, 2.7 Hz, 1 H), 4.65 (ddd, *J =* 10.0, 3.2, 2.0 Hz, 1 H), 4.58 (dd, *J* = 12.4, 2.0 Hz, 1 H), 4.40 (dd, *J* = 12.4, 3.2 Hz, 1 H), 3.96 (t, *J* = 6.6 Hz, 2H), 3.06 (dd, *J* = 16.2, 2.7 Hz, 1 H), 2.76 (dd, *J =* 16.2, 4.3 Hz, 1 H), 1.76 (dq, *J* = 8.1, 6.6 Hz, 2H), 1.51 - 1.29 (m, 4H), 0.91 (t, *J* = 7.1 Hz, 3H). ¹³C NMR (100 MHz, CDCl₃) δ (ppm): 169.3, 165.4, 159.3, 129.6, 127.7, 120.9, 119.4, 113.7, 75.3, 69.0, 68.4, 64.4, 34.5, 29.1, 28.4, 22.6, 14.2. HPLC-MS (ES+): Gradient MeCN/H₂O 10:90 to 100:0 (5 min), tr: 4.9 min, [M+H]⁺ = 304.0. Elemental analysis (AE): calculated for C₁₇H₂₁NO₄: C, 67.31%; H, 6.98%; N, 4.62%; found: C, 67.08%; H, 6.88%; N, 4.65%. Optical rotation: [α]_{D} = -82 (c = 1, CHCl₃).

### Example 6. Synthesis of 2-(3-(2-morpholinoethoxy)phenyl)-7,7a-dihydro-3aH-pyrano[3,4-d]oxazole-6(4H)-one (IQM11PS06)

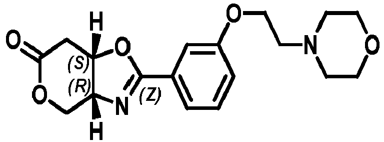

Obtained following the process described using 3-(2-morpholinoethoxy)benzoic acid (overall yield: 60%).

¹H NMR (300 MHz, CDCl₃) δ (ppm): 7.46 (m, 1 H), 7.41 (m, 1 H), 7.25 (m, 1 H), 7.03 (m, 1 H), 5.22 (ddd, *J* = 9.9, 4.3, 2.7 Hz, 1 H), 4.64 (ddd, *J* = 10.0, 3.1, 1.9 Hz, 1 H), 4.59 (dd, *J* = 12.3, 2.0 Hz, 1 H), 4.42 (dd, *J* = 12.4, 3.2 Hz, 1 H), 4.12 (t, *J* = 8.0, 2H), 3.72 (m, 4H), 3.10 (dd, *J =* 16.2, 2.8 Hz, 1 H), 2.80 (m, 2H), 2.60 (m, 2H). ¹³C NMR (75 MHz, CDCl₃) δ (ppm): 169.4, 165.5, 159.0, 129.9, 128.1, 121.5, 119.6, 114.2, 75.6, 69.1, 67.3, 66.4, 64.6, 58.0, 54.5, 34.7, 29.3. HPLC-MS (ES+): Gradient MeCN/H₂O 10:90 to 100:0 (5 min), tr: 1.7 min, [M+H]⁺ = 347.0. Elemental analysis (AE): calculated for C₁₈H₂₂N₂O₅: C, 62.42%; H, 6.40%; N, 8.09%; found: C, 62.58%; H, 6.69%; N, 7.85%. Optical rotation: [α]_{D} = -54 (c = 1,1, CHCl₃).

### Pharmacological activity

In this invention, the relation between the expression of the transcription factor HIF-2α and the population of *CSCs* was studied using the tumour mammospheres model and its functional regulator SOX-2, thereby establishing the foundations for pharmacological characterization of this cell subpopulation based on the pathways activated by these transcription factors.

In particular, various compounds of formula **I**, represented by the compound IQMPS04, were characterised for their action on cell differentiation in breast cancer models, measuring both the reduction of the *CSCs* (mammospheres) subpopulation and the repression of the HIF transcription factors, particularly HIF-2α, and SOX-2, as an anti-tumour strategy.

### Example 7. IQM11PS04 significantly inhibits in the HRE-LUC test in the MDA-MB-231 line

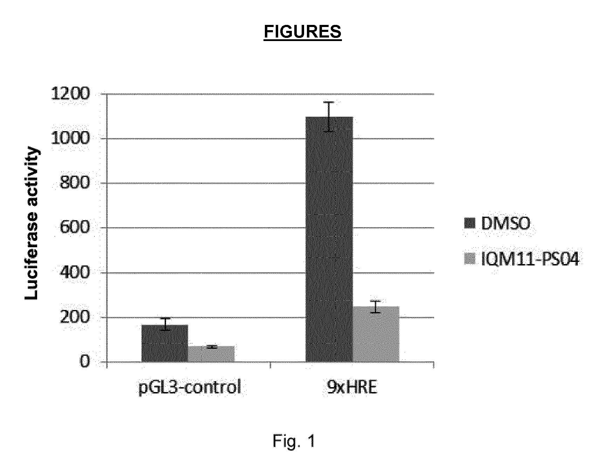

In order to identify direct inhibitors of HIF activity, a breast tumour line MCF7 was first constructed containing the deoxyribonucleic acid sequences (DNA) of the region located between the positions -985 and -951 of the human vascular endothelium growth factor (hVEGF), repeated nine times (X9) and fused to the luciferase reporter gene (LUC) (Moreno-Manzano V, et al (2010) JBC 285 (2) 1333-1342). This plasmid called 9XHRE-LUC, kindly given by Dr Manuel Ortiz de Landazuri of the La Princesa Hospital of Madrid, was prepared using a commercial kit (Endofree Maxi-Prep; Qiagen, Inc., Valencia, CA). Transfections were performed with Lipofectamine (Invitrogen) according to the manufacturer's instructions. Figure 1 shows the inhibition in the expression of luciferase in the presence of increasing concentrations of IQM11PS04.

### Example 8. IQM11PS04 significantly inhibits the induction of SOX2

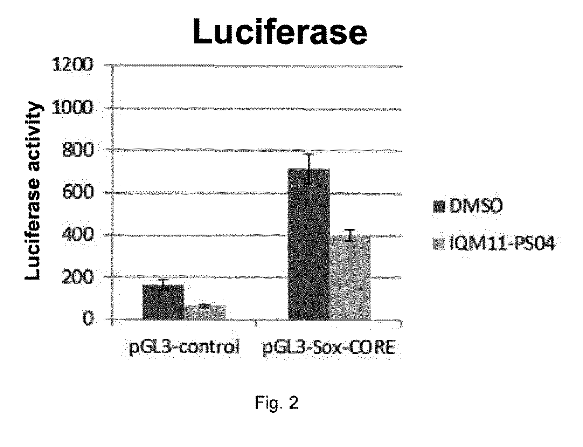

In order to identify whether the direct inhibitors of mammosphere formation and HIF activity inhibited the transcriptional activation of *SOX2,* reported in breast cancer CSCs (Leis, O., et al (2012), Oncogene, 31, 1354-1365), breast tumour lines were constructed, transiently transfected with the promoter region of enhancer R1 of SOX-2 by Lipofectamine (Invitrogen) according to the manufacturer's instructions. Their induction on cultivating the cells in suspension was compared and the inhibition of this activation was measured on treating the cells with the inhibitor IQM11PS04. Figure 2 shows the inhibitory activity of compound IQM11PS04 on the expression of the promoter region of enhancer R1 of SOX-2 in the breast tumour line MDA_MB-436 / HRE9X-LUC.

### Example 9. IQM11PS04 significantly inhibits the formation of tumour mammospheres

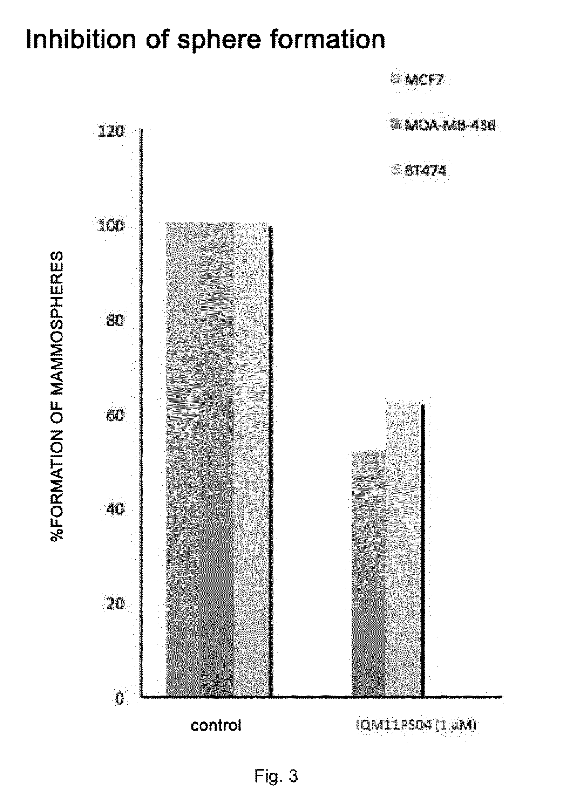

The formation of spheres derived from breast tumours has been described as an effective method for enrichment of cell cultures in CSCs because of the higher ability of cells derived from these culture in generating tumours in vivo when transplanted in immunocompromised mice (Ponti D, et al., (2005) Cancer Res 65(13):5506-11, 34). A directly proportional relationship is therefore established between the percentage of the *CSCs* subpopulation in the culture and the ability to form spheres. Tumour cell lines growing in suspension are used in these tests in order to enrich the culture in tumour stem cells. CSCs are the only cells capable of proliferating in these conditions, giving rise to structures named spheres. In fact, the cells that grow in suspension acquire the properties of tumour stem cells and are used in the assays used in this patent for identifying drugs effective against them. In principle, compounds that affect tumour stem cells should slow their growth and potentially cause their death. This assay is tuned for breast cancer cell lines according to Leis, O., et al (2012), Oncogene, 31, 1354-1365. Cells are seeded at a density of 1,000 cells/mL on 6-well plates, previously treated with poly-HEMA (poly(2-hydroxyethyl methacrylate)), Sigma-Aldrich) to prevent the cells adhering to the plastic of the plate. A DMEM high glucose medium supplemented with F12 (Nutrient Mixture F-12 HAM, Sigma), B27 (B27 Supplement 50X, Gibco 17504-044) and growth factors, EGF and bFGF is used for culture. Methylcellulose at a final concentration of 0.5% was added to increase the viscosity of the culture medium. Cells were incubated for 7 days at 37° C, adding growth factors (20 ng/mL of each factor), every two days and counted under a microscope. The inhibitor IQM11PS04 showed significant inhibitory ability in the formation of mammospheres when added at a dose of 1 µM in the final stage of sphere formation in the same cell lines used for the cell cytotoxicity test with MTT. The reduction both in sphere numbers and in their size, in addition to a change in cell morphology to less spherical and regular was observed in those treated with the inhibitor compared to those treated with DMSO (Fig. 1). A reduction in the number of spheres and less defined morphology and smaller diameter was observed in cells treated with the inhibitor. Figure 3 shows the activity of IQM11PS04 on the formation of mammospheres in different cell lines.

### Example 10. IQM11PS04 does not affect cell viability in cultured tumour lines MCF-7, BT-474 and MDA-MB-436

In order to determine the toxicity of the compound, cell viability assays were performed with MTT on breast tumour lines MCF-7, BT-474 and MDA-MB-436. The HIF-2α inhibitor IQM11PS049 used at the normal dose (1µM) as well as at a tenfold higher dose exerted significant action on HIF-2α and did not cause a significant increase in the percentage of cell death in treated cultures (Fig. 3). The fact that cell viability was not affected at a high concentration reflects the low toxicity of the compound at the dose required for its effectiveness in functional assays.

### Example 11. Summary of the activities presented by compounds of formula I on the formation of spheres

Other compounds of formula **I** were tested following the methods described in examples 7 to 10. The following table (Table 2) includes a summary of the results obtained.

**Table 2: summary of the activity shown by the compounds on mammosphere formation. All the compounds were obtained by the method described in examples 1 to 6. [% viab. approx. approximate viability percentage; % inhib., inhibition percentage].**

| **COMPOUND** | **TOXICITY 1uM** | | | **HIF** | **ACTIVITY IN SPHERE TEST** | | |
|---|---|---|---|---|---|---|---|
| | **MCF-7** | **BT-474** | **MDA-MB-436** | **HRE-LUC** | *% sphere formation* | | |
| | **(% viab. approx .)** | **(% viab. approx .)** | **(% viab. approx .)** | **(% inhib. 5µM)** | **MCF-7** | **MDA-MB-436** | **BT474** |
| IQM11PS01 | 115 | 97 | - | - | 0 | - | 2 |
| IQM11PS02 | 99 | 55 | - | - | 0 | - 4 | 4 |
| IQM11PS03 | 98 | 97 | - | - | 0 | 88 | 12 |
| IQM11PS04 | 109 | 89 | - | 92 | 0 | | 1 |
| IQM11PS05 | 92 | 168 | - | 96 | 0 | | 8 |
| IQM11PS06 | 97 | 86 | - | - | 20 | 79 | 27 |

## Claims

1. A compound of formula **I**: 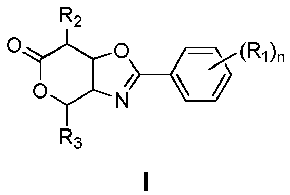 or a salt thereof where:
each R₁ independently represents alkyl C₁₋₁₂, alkenyl C₂₋₁₂, alkynyl C₂₋₁₂, alkyl Cy₁-C₁₋₄, alkenyl Cy₁-C₂₋₄, alkynyl Cy₁-C₂₋₄, -OR₄, -SR₄ or -NR₅R₅, where each alkyl C₁₋₁₂, alkenyl C₂₋₁₂, alkynyl C₂₋₁₂, alkyl Cy₁-C₁₋₄, alkenyl Cy₁-C₂₋₄ and
alkynyl Cy₁-C₂₋₄ are independently substituted by one or more halogen, -O(alkyl C₁₋₄), -S(alkyl C₁₋₄), -NH(alkyl C₁₋₄) or -N(alkyl C₁₋₄)₂ group;
R₂ represents hydrogen, alkyl C₁₋₄, -OR₄, -SR₄ or -NR₅R₅;
R₃ represents hydrogen or alkyl C₁₋₄;
n represents from 0 to 5;
each R₄ independently represents hydrogen, alkyl C₁₋₁₂, alkenyl C₂₋₁₂, alkynyl C₂₋₁₂, alkyl Cy₁-C₁₋₄, alkenyl Cy₁-C₂₋₄ or alkynyl Cy₁-C_{2-4;}
each R₅ independently represents hydrogen, alkyl C₁₋₄, -COR₆, -CONR₆R₆, -CO₂R₆ or -SO₂R₆;
or two R₅ on the same N atom may be bound forming a 5 or 6 member ring with the N atom, which may additionally contain one or two heteroatoms selected from N, S and O and optionally substituted by one or more alkyl C₁₋₄ groups;
each R₆ independently represents hydrogen, alkyl C₁₋₁₂, alkenyl C₂₋₁₂, alkynyl C₂₋₁₂, alkyl Cy₁-C₁₋₄, alkenyl Cy₁-C₂₋₄, alkynyl Cy₁-C_{2-4;}
Cy₁ represents phenyl or a saturated, partially unsaturated or aromatic 5 or 6 member heterocyclic ring linked to the rest of the molecule through any available C or N atom, each of which can be optionally fused to a saturated,
partially unsaturated or aromatic 5 or 6 member heterocyclic ring, where Cy₁ may contain from 1 to 4 heteroatoms in total selected from N, O and S, where one or more of the available C or N atoms in Cy₁ are optionally oxidised forming CO, SO or SO₂ groups and where Cy₁ is optionally substituted by one or more R7;
R₇ represents alkyl C₁₋₄, halogen, -CN, -OR₈, -SR₈, -NR₈R₈; and
each R₈ independently represents hydrogen or alkyl C₁₋₄.

2. The compound of claim 1 wherein R₁ independently represents alkyl C₁₋₁₂, alkyl Cy₁-C₁₋₄, -OR₄, -SR₄ or -NR₅R₅.

3. The compound of any of the claims 1 or 2 wherein R₂ represents hydrogen or alkyl C₁₋₄.

4. The compound of any of the claims 1 to 3 wherein R₃ represents hydrogen or methyl.

5. The compound of any of the claims 1 to 4 wherein n represents from 0 to 2.

6. The compound of any of the claims 1 to 5 wherein each R₄ independently represents hydrogen, alkyl C₁₋₁₂ or alkyl Cy₁-C₁₋₄.

7. The compound of any of the claims 1 to 6 wherein:
each R₅ independently represents hydrogen or alkyl C₁₋₄ or
or two R₅ groups on the same N atom may be bound forming a 5 or 6 member ring with the N atom, which may additionally contain one or two heteroatoms selected from N, S and O and optionally substituted by one or more alkyl C₁₋₄ groups.

8. The compound of any of the claims 1 to 7 wherein each R₆ independently represents hydrogen, alkyl C₁₋₁₂ or alkyl Cy₁-C₁₋₄, and preferably hydrogen or alkyl C₁₋₁₂, and more preferably hydrogen or methyl.

9. The compound of any of the claims 1 to 8 wherein Cy₁ represents phenyl or a saturated, partially unsaturated or aromatic 5 or 6 member heterocyclic ring bound to the rest of the molecule through any available C or N atom, where Cy₁ may contain 1 or 2 heteroatoms in total selected from N, O and S, and where Cy₁ is optionally substituted by one or more R₇, preferably where Cy₁ is optionally substituted by one or two R₇.

10. The compound of any of the claims 1 to 9, wherein:
each R₁ independently represents alkyl C₁₋₁₂, alkyl Cy₁-C₁₋₄, -OR₄, -SR₄ or -NR₅R₅, preferably -OR₄, -SR₄ or -NR₅R₅, and still more preferably -OR₄; and
n represents from 0 to 2, and more preferably 1 or 2.

11. A compound of formula **I** of any of the claims 1 to 10 for its use in therapy.

12. A pharmaceutical composition comprising a compound of formula **I** of any of the claims 1 to 10 and at least one pharmaceutically acceptable excipient.

13. A compound of formula **I** of any of the claims 1 to 10 or a pharmaceutically acceptable salt thereof for its use in the treatment of cancer.

14. The compound of claim 13 for the treatment of breast cancer, brain cancer, head cancer, neck cancer, liver cancer, cervical cancer, colorectal cancer, endometrial cancer, gastric cancer, lung cancer, pancreatic cancer, ovarian cancer, prostate cancer and renal cancer.

15. Process for obtaining a compound of formula **I** of any of the claims 1 to 10 that comprises:
(a) making a compound of formula **II** cyclic in the presence of an acid 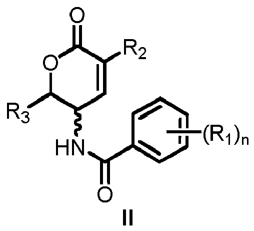 where R₁, R₂, R₃ and n have the previously described meanings; or
(b) transforming, in one or several stages, a compound of formula **I** into another compound of formula **I**.
